# EUROPÄISCHE PATENTSCHRIFT

(11) **EP 2 764 848 B1**
(45) Veröffentlichungstag und Bekanntmachung des Hinweises auf die Patenterteilung: **31.08.2016**
(21) Anmeldenummer: 13154638.4
(22) Anmeldetag: 08.02.2013
(51) Int. Cl.: A61F 2/30, A61F 2/34, A61B 17/80

(54) **Endoprothese für den Teilersatz des menschlichen Beckenknochens**
Endoprosthetic for the partial replacement of the human pelvic bone
Endoprothèse pour le remplacement partiel de l'os du bassin humain

(43) Veröffentlichungstag der Anmeldung: 13.08.2014
(73) Patentinhaber: WALDEMAR LINK GmbH & Co. KG, 22339 Hamburg (DE)
(72) Erfinder: Link, Helmut D., 22397 Hamburg (DE)
(74) Vertreter: Raffay & Fleck

(56) Entgegenhaltungen:
- WO-A1-88/01491
- CN-A- 102 048 598
- DE-C1- 19 700 160
- DE-C1- 19 747 357
- DE-T2- 69 729 265
- US-A1- 2003 171 818
- US-A1- 2003 212 459
- US-A1- 2006 116 775

## Beschreibung

Die vorliegende Erfindung betrifft eine Endoprothese für den Teilersatz des menschlichen Beckenknochens im Bereich des Acetabulum sowie des Os Ilium (Darmbeins) mit den Merkmalen des Oberbegriffs des Anspruches 1.

Es ist bekannt, dass aufgrund von Verschleiß, Knochenschwäche (z.B. Osteoporose) oder durch andere Erkrankungen hervorgerufene Knochenveränderungen, beispielsweise aufgrund von Tumorerkrankungen, im Beckenbereich die dortige natürliche Knochenstruktur derart in Mitleidenschaft geraten und sich verändern kann, dass die Knochenstatik nicht mehr gegeben ist und/oder die Gelenkfunktion, insbesondere des Acetabulum, nicht mehr gegeben ist. Auch in Folge von Unfällen und komplizierten Frakturen kann es hier zu entsprechenden Schädigungen und Ausfällen kommen.

Häufig sind dabei solche Fälle, in denen nur der beckenseitige Teil des Hüftgelenkes, das Acetabulum und seine unmittelbar umgebenden Bereiche, in Mitleidenschaft gezogen sind. In diesen Fällen werden Hüftendoprothesen eingesetzt, bei denen in das vorhandene, ansonsten hinsichtlich seiner Knochenstruktur und Tragfähigkeit noch intakte Becken des Patienten anstelle des defekten natürlichen Acetabulum eine künstliche Gelenkpfannenkomponente eingesetzt und im Beckenknochen verankert wird.

In solchen Fällen jedoch, in denen weitere Teile des Beckenknochens defekt und in Mitleidenschaft gezogen sind, müssen weiträumigere Ersetzungen vorgenommen werden.

In der CN 102048598 A ist ein Beckenteilersatz gezeigt, mit dem bei einer besonders massiven Schädigung des natürlichen Beckenknochens vollständig das Os Ilium nebst daran angeformtem Acetabulum und auch zumindest ein Teil, in einer Ausgestaltungsvariante sogar insgesamt, das Os Ischium ersetzt werden.

Die Endoprothese der Erfindung geht indes jedoch aus von weniger umfangreichen und gravierenden Ersetzungen, betrifft vielmehr solche Endoprothesen für den Teilersatz des menschlichen Beckenknochens, die den Bereich des Acetabulum sowie einen Teil des Os Ilium abdecken.

Vergleichbare Endoprothesen, die neben einem Ersatz des Acetabulum sich zumindest auch über den Bereich des Os Ilium hin erstrecken, sind aus dem Stand der Technik bekannt. So ist in der DE 697 29 265 T2 ein modulares Verstärkungssystem die Gelenkpfanne des Hüftgelenkes bekannt. Hier sind einzelne flächige Anbaumodule vorgesehen, die mit einem Acetabulum-Ersatz verbunden werden können und von denen sich ausgesuchte jedenfalls auch über das Os Ilium erstrecken können. Die dort gezeigten Anbauelemente sind lösbar mit dem Acetabulum verbunden und dienen dort nicht etwa einem Ersatz von Knochendefekten am Os Ilium, sie sollen vielmehr die Festigkeit der Verankerung des Acetabulum-Ersatzes erhöhen. Ähnliche mit weiteren Elementen außerhalb des Acetabulums liegende Abschnitte des Beckenknochens, insbesondere des Os Ilium überdeckende Lösungen zeigen die WO 88/01491 A1, welche die Merkmale des Oberbegriffs von Anspruch 1 zeigt, und die US 2003/0171818 A1. Auch bei den in diesen beiden Schriften gezeigten Lösungen dienen diese weiteren Elemente lediglich der sicheren Verankerung des Acetabulumersatzes, nicht jedoch einem gezielten Ersatz des Os Ilium im Bereich eines weiteren Defekts außerhalb des Acetabulums.

Auch die DE 197 47 357 C1, in der eine Weiterbildung der in der DE 197 00 160 C1 offenbarten Becken-Teilendoprothese beschrieben wird, zeigt eine Becken-Teilendoprothese, bei der lösbar mit einem Acetabulum-Ersatz verbunden eine über das Os Ilium geführte Brücke, dort als Überbrückungsstück bezeichnet, angefügt werden kann für eine Festlegung der so entstandenen Gesamtprothese auch an diesem Beckenteil. Dieses zusätzliche Element dient einer Stabilisierung der Teilendoprothese auch bei durchtrenntem Beckenknochen, unterstützt ein Verspannen der Fraktur, welche durch einen in die Öffnung zwischen Os Ilium, Os Ischium und Os Pubis eingreifenden Haken bewirkt wird und dort eine die Fraktur zusammenziehende Zugkraft ausübt.

Die bekannten letztgenannten beiden Lösungen aus dem Stand der Technik dienen insbesondere nicht der Überbrückung bzw. Reparatur eines weitergehenden Knochendefektes, der sich auch in das Os Ilium hinein erstreckt, hier beispielsweise aufgrund schwerer Verletzungen, insbesondere aber bei pelvinen Knochentumoren entstehen kann.

Hier soll also mit der Erfindung eine Endoprothese für den Teilersatz des menschlichen Beckenknochens der eingangs genannten Art angegeben werden, mit der mit einer einzigen Prothese ein Knochenersatz und eine Stabilisierung im Bereich sowohl des Acetabulum als auch für defekte Abschnitte des Os Ilium erreicht werden kann, welche sich mit anderen Worten also eignet mit einem einzigen Teil auch bei größeren Knochendefekten im Beckenbereich die Stabilität und Tragfähigkeit dieses Skelettabschnittes wieder herzustellen und die Gelenkigkeit und Bewegbarkeit in diesem Bereich zu erhalten bzw. neu zu gestalten.

Diese Aufgabe wird erfindungsgemäß dadurch gelöst, dass sich der zweite Abschnitt ausgehend von dem ersten Abschnitt über den Rand der ersten teilsphärischen Vertiefung abgeflacht weiter erstreckt und einstückig mit dem ersten Abschnitt verbunden ist und dass ferner in dem zweiten Abschnitt eine zweite teilsphärische Vertiefung eingebracht ist. Diese zweite teilsphärische Vertiefung ist dabei in gleicher Richtung eingebracht wie diejenige, die als Ersatz für das Acetabulum dient, ist also bei Aufsicht auf die erfindungsgemäße Endoprothese aus einer Richtung in gleicher Krümmungsrichtung verlaufend, mit einer Aufsicht von oben (also auf die nach dem Einsetzen der Endoprothese in den menschlichen Beckenknochen auf der Oberfläche sichtbare Seite) konkav gekrümmt, bei einer Aufsicht von der gegenüberliegenden Rückseite her konvex.

Durch die erfindungsgemäße Ausbildung einer einstückigen Endoprothese, also einer einstückigen Verbindung zwischen dem ersten Abschnitt mit der ersten teilsphärischen Vertiefung und dem zweiten Abschnitt mit der zweiten teilsphärischen Vertiefung ist diese Prothese in höchstem Maße stabil, können die auf dieser Prothese durch den Bewegungsapparat hervorgerufenen Belastungen sicher aufgenommen und zuverlässig in Richtung der Verankerungsbereiche der Prothese im verbleibenden Beckenknochen abgeführt und von dort auf das natürliche Skelett übertragen werden.

Die erfindungsgemäßen, im zweiten Abschnitt vorgesehene zweite sphärische Vertiefung, die in Einbaulage am kranialen Ende der Endoprothese liegt, wohingegen die das Acetabulum ersetzende erste teilsphärische Vertiefung am kaudalen Ende der Endoprothese liegt, dient einer guten und sicheren Abstützung der Endoprothese in diesem Abschnitt am verbleibenden Restknochen des Os Ilium. Darüber hinaus können entsprechende zur zweiten teilsphärischen Vertiefung komplementär gebildete Abstützflächen am verbleibenden Knochenmaterial des Os Ilium einfach ausgebildet werden.

Der Begriff "teilsphärisch", so wie er in der vorliegenden Beschreibung Verwendung findet, besagt, dass die entsprechenden Vertiefungen in ihrer Oberflächenform jedenfalls in einem Abschnitt auf einem Kugelabschnitt, also einer Teilsphäre, liegen und entsprechend gebildet verlaufen.

Mit Vorteil liegt auf der der ersten teilsphärischen Vertiefung gegenüberliegenden Seite der zweiten teilsphärischen Vertiefung und an dieser einstückig angeformt in einem Winkel abstehender, verbreiterter und abgeflachter Rand. Mit diesem Rand, der in seiner genauen Formgebung idealer Weise dem Formverlauf der Oberfläche des Os Ilium nachempfunden ist, kann sich die Endoprothese im Bereich um die zweite teilsphärische Vertiefung herum an diesem Knochenabschnitt abstützen, gibt so eine weitere statisch günstige Kraftübertragung zu dem und Krafteinleitung in das Os Ilium.

Mit Vorteil sind in dem ersten und/oder in dem zweiten Abschnitt einstückig angeformte, sich nach außen erstreckende Laschen vorgesehen, die jeweils wenigstens eine Durchlassöffnung zum Durchführen von Befestigungsmitteln, insbesondere Knochenschrauben, aufweisen. Grundsätzlich kann hierbei eine einzige derartige Lasche für die Verankerung der Endoprothese genügen, bevorzugt werden jedoch wenigstens zwei oder gar mehr derartige Laschen. Diese Laschen müssen nicht zwingend in einer Ebene gerade geführt verlaufen, sie können auch Abwinklungen aufweisen, um aus verschiedenen Winkeln in den verbleibenden Knochen des Patienten Verankerungen, insbesondere in Form von Knochenschrauben, einzubringen und die erfindungsgemäße Endoprothese festzulegen.

Weitere Durchlassöffnungen zum Durchführen eines Befestigungsmittels können in der ersten und/oder der zweiten teilsphärischen Vertiefung vorgesehen sein. Insbesondere in der ersten teilsphärischen Vertiefung sollten dabei entsprechende Durchlassöffnungen vorgesehen sein, um in dem im späteren Einsatz der Endoprothese besonders beanspruchten Bereich des Acetabulum-Ersatzes die Endoprothese festzulegen.

Bei der erfindungsgemäßen Endoprothese kann insbesondere in einem Übergangsbereich zwischen dem ersten und dem zweiten Abschnitt und zwischen der ersten und der zweiten teilsphärischen Vertiefung angeordnet, ansonsten vom Material der einstückig gebildeten Endoprothese umgeben ein Durchbruch vorgesehen sein. Ein solcher Durchbruch bietet eine Materialersparnis und reduziert dadurch das Gewicht der Endoprothese. Darüber hinaus gibt der Durchbruch dem Operateur die Möglichkeit beim Ausrichten und Einsetzen der Endoprothese durch den Durchbruch auf den darunter liegenden Bereich zu schauen und sich so im Operationsfeld besser orientieren zu können. Die Materialabschnitte, die neben dem Durchbruch den ersten und den zweiten Abschnitt verbinden, dienen dann noch der Überbrückung eines im Knochen des Patienten bestehenden Defekts, den es mit der Endoprothese zu überbrücken gilt.

Insbesondere ist bei der erfindungsgemäßen Endoprothese die zweite teilsphärische Vertiefung so gebildet, dass der teilsphärisch ausgebildete und aus der Fläche der Endoprothese in die Richtung gezogene Bereich, in der auch die erste teilsphärische Vertiefung sich hinein erstreckt, um einen der ersten teilsphärischen Vertiefung entfernt gelegen gegenüberliegenden Bereich des zweiten Abschnittes herum gebildet ist, also an einem kranialen Ende der Endoprothese. Der gemäß der oben genannten vorteilhaften Weiterbildung ausgebildete Durchbruch liegt dann insbesondere zwischen diesen Bereichen der ersten und der zweiten teilsphärischen Vertiefung. Hier wird einerseits Material der Endoprothese gespart, andererseits kann fertigungstechnisch das Material zum Ausbilden der zweiten teilsphärischen Vertiefung durch Aufstoßen dieses Durchbruches aus der Fläche der Endoprothese heraus erhalten werden.

Bei der erfindungsgemäßen Endoprothese können im Bereich der ersten teilsphärischen Vertiefung auf der konkav gekrümmten Seite des Materials dellenartige Ausnehmungen vorgesehen sein. Diese dienen bei der Festlegung des Gelenkpfanneneinsatzes in die Endoprothese und einer verbesserten Verbindung, beispielsweise mittels Kleber oder Zement.

Ebenso können im Bereich der zweiten teilsphärischen Vertiefung auf der konvex gekrümmten Seite des Materials dellenartige Ausnehmungen vorgesehen sein. Auf diese können einer verbesserten Verankerung der Endoprothese am residuellen Knochenmaterial in diesem Bereich dienen, beispielsweise bei einer zusätzlichen Festlegung der Endoprothese mittels Knochenzement.

Mit Vorteil ist in dem Bereich, in dem sich an die erste teilsphärische Ausnehmung und deren Rand der zweite Abschnitt anschließt, eine das kraneale Pfannendach nachbildende Struktur vorgesehen. Das kraneale Pfannendach im natürlichen Hüftgelenk ist wichtig, um Hüftluxationen bei Bewegungen zu vermeiden. Da bei den mit der erfindungsgemäßen Endoprothese zu behandelnden Knochendefekten gerade auch dieser Teil des natürlichen Knochens soweit verschlissen oder verändert ist, dass seine Funktion nicht mehr gegeben ist, muss auch dieser für eine zukünftige Funktion des gebildeten künstlichen Gelenkes nachgebaut bzw. nachgebildet werden. Hier bietet es sich an, diese Struktur bei der einstückigen Ausbildung der erfindungsgemäßen Endoprothese mit zu integrieren und in dieser Prothese vorzusehen.

Die erfindungsgemäße Endoprothese ist insbesondere aus einem Metall, welches vorzugsweise hoch poliert ist, gebildet und kann dabei mit besonderem Vorteil aus Titan oder einer Titanlegierung bestehen. Derartige Materialien haben sich in der Endoprothetik bewährt als ausreichend stabil zur Übertragung der auflastenden Kräfte einerseits und andererseits hinsichtlich ihrer Biokompatibilität.

Die erfindungsgemäße Endoprothese wird insbesondere für die betroffenen Patienten je nach individuellem Befund maßgefertigt. Hierzu werden typischerweise mit bekannten bildgebenden Verfahren (z.B. Computertomographie) Aufnahmen des verschlissenen Beckenabschnittes des zu behandelnden Patienten angefertigt, wird anhand dieser Aufnahme ein Modell gebildet, z.B. mit dem Verfahren des sogenannten Rapid-Prototyping, und wird anhand dieses Modells die Planung einer passenden Endoprothese vorgenommen. Die Herstellung einer solchen Endoprothese kann dabei insbesondere einen Schritt des Gießens in einer Gießform und des weiteren Bearbeitens des Gussteils durch z.B. Schleifen und Polieren umfassen. Möglich ist es aber auch, dass zunächst eine Rohform hergestellt und dann durch Kaltumformen wie beispielsweise Kaltstanzen, oder aber auch durch Warmumformen, wie beispielsweise Schmieden, ein Rohling der Endoprothese hergestellt wird, der dann durch weiteres Bearbeiten, beispielsweise Schleifen und Polieren, fertig gestellt wird.

Weitere Vorteile und Merkmale der Erfindung ergeben sich aus der nachfolgenden Beschreibung eines Ausführungsbeispiels anhand der beigefügten Figuren. Dabei zeigen:
- Fig. 1: in einem ersten Ausführungsbeispiel eine erfindungsgemäße Endoprothese für den Teilersatz des menschlichen Beckenknochens in einer Ansicht von oben, d.h. von der in Implantationsstellung dem Knochen abgewandten Seite her;
- Fig. 2: die Endoprothese aus Fig. 1 in einer Ansicht von unten;
- Fig. 3: eine Ansicht von unten auf ein zweites Ausführungsbeispiel einer erfindungsgemäßen Endoprothese;
- Fig. 4: ein drittes Ausführungsbeispiel einer erfindungsgemäßen Endoprothese in einer Implantationsstellung im Becken;
- Fig. 5: das Ausführungsbeispiel aus Fig. 4 in Implantationsstellung im Becken, gezeigt aus einem anderen Blickwinkel; und
- Fig. 6: eine Veranschaulichung einer Prothesenplanung anhand eines dem defekten Beckenknochen eines Patienten nachgebildeten Modells.

In den Figuren sind verschiedene Ausführungsbeispiele erfindungsgemäßer Endoprothesen für den Teilersatz des menschlichen Beckenknochens in unterschiedlichen Ansichten dargstellt. Die Figuren zeigen dabei auch die Implantationslage solcher Endoprothesen sowie eine Veranschaulichung für die Planung einer erfindungsgemäßen Endoprothese anhand eines Modells eines defekten Beckenknochens eines Patienten. Dabei sind die Figuren keinesfalls beschränkend, sie dienen lediglich zusammen mit der nachfolgenden Beschreibung der Ausführungsbeispiele der Erläuterung und dem besseren Verständnis der Erfindung.

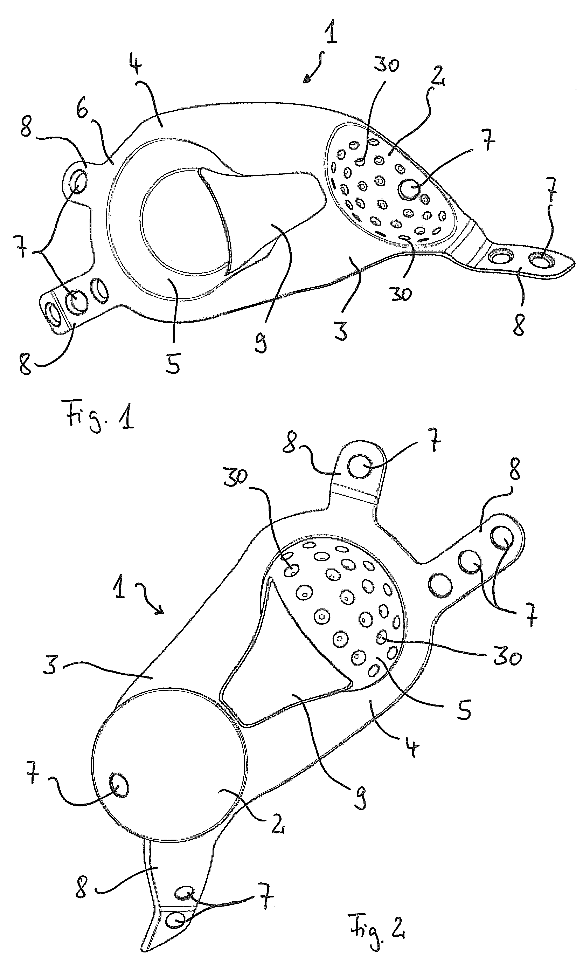

In den Figuren 1 und 2 ist aus verschiedenen Ansichten, einmal in einer Ansicht von oben, d.h. auf die in Implantationsstellung dem Knochen abgewandte, freiliegende Oberfläche der Endoprothese (Fig. 1), einmal in einer Ansicht von unten, d.h. auf die in Implantationsstellung an dem Knochen anliegende Oberfläche der Prothese (Fig. 2), ein erstes Ausführungsbeispiel für eine erfindungsgemäße Endoprothese für den Teilersatz des menschlichen Beckenknochens gezeigt. Diese Endoprothese ist in dem ersten Ausführungsbeispiel mit der Bezugsziffer 1 bezeichnet. Sie weist eine erste teilsphärische Vertiefung 2 in einem ersten Abschnitt 3 auf, welche erste teilsphärische Vertiefung 2 als Ersatz des Acetabulums in dem zu versorgenden Beckenknochen des menschlichen Patienten dient.

In einem zweiten Abschnitt 4, der im implantierten Zustand der Endoprothese 1 am Os Ilium des Patienten anliegt, ist eine zweite teilsphärische Vertiefung 5 vorgesehen. Um diese zweite teilsphärische Vertiefung 5 ist ein abgeflachter Rand 6 gebildet, der diese in einem weiten Teil umgibt. Ausgehend von diesem Rand 6 einerseits und angrenzend an die erste teilsphärische Vertiefung 2 andererseits sind mit Befestigungsöffnungen 7 versehene Befestigungslaschen 8 an der Endoprothese 1 angeformt, mittels derer diese an umgebenden Knochenmaterial des Beckens festgelegt werden kann, indem Knochenschrauben durch die Befestigungsöffnung 7 hindurch geführt und im Beckenknochen fixiert werden. Eine weitere Befestigungsöffnung 7 ist in der ersten teilsphärischen Vertiefung 2, die das Acetabulum nachbildet, zu erkennen. Auch diese Befestigungsöffnung 7 in der teilsphärischen Vertiefung 2 dient der Fixierung, insbesondere der Primärfixierung der Endoprothese 1, hier in dem besonders belasteten Bereich des Acetabulums, welches die künstliche Gelenkpfanne aufnimmt.

Zu erkennen ist auch, dass die Befestigungslaschen 8 nicht gerade und eben verlaufen, sondern abgewinkelt und gekröpft. Der Verlauf der Befestigungslaschen 8 ist dabei nach den anatomischen Verhältnissen der Oberfläche des Beckenknochens des mit der Endoprothese zu versorgenden Patienten gewählt und gebildet.

Die Endoprothese 1 ist einstückig gebildet, insbesondere mit einer einstückigen Verbindung zwischen den beiden Abschnitten 3 und 4. Neben einem diese Abschnitte einstückig verbindenden Bereich ist jedoch auch ein Durchbruch 9 vorgesehen, der hier eine die Fläche der Endoprothese 1 durchragende Öffnung bildet. Diese Durchbruch führt zu einer Material- und Gewichtsersparnis der Endoprothese 1 und erlaubt dem Operateur während des Ausrichtens und Setzens der Endoprothese 1 einen Durchblick durch diese auf darunter liegende Bereiche, was ihm eine verbesserte Orientierung im Operationsfeld ermöglicht.

Auf der konkaven, auf der Oberseite der Endoprothese 1 gelegenen Innenfläche der teilsphärischen Vertiefung 2 und auf der konvexen, auf der Unterseite der Prothese gelegenen Außenfläche der zweite teilsphärischen Vertiefung 5 sind jeweils in regelmäßigem Muster angeordnete, dellenartige Ausnehmungen 30 angebracht. Diese sind den Figuren 1 bzw. 2 gut zu erkennen. Diese dellenartigen Ausnehmungen 30 dienen einer Vergrößerung der jeweiligen Oberfläche für eine bessere Verbindung der so in ihrer Oberfläche vergrößerten Bereiche mit einer in die konkave Aufnahme der ersten teilsphärischen Vertiefung 2 eingesetzten künstlichen Gelenkpfanne für eine Fixierung derselben mit einem Kleber oder Zement bzw. für eine Befestigung der zweiten teilsphärischen Vertiefung 5 am Beckenknochen des Patienten im Bereich des Os Ilium.

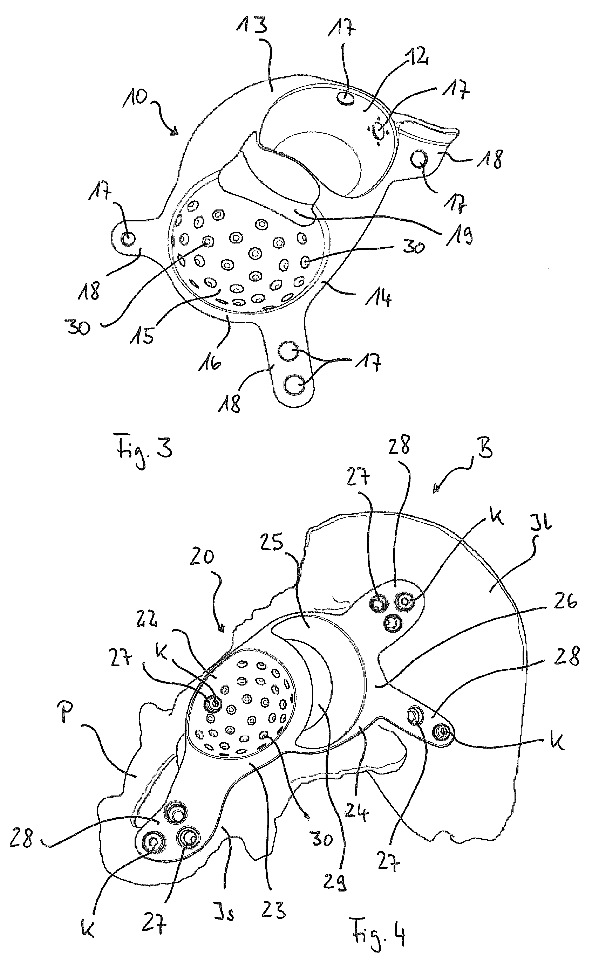

In Fig. 3 ist ein zweites Ausführungsbeispiel einer erfindungsgemäßen Endoprothese 10 gezeigt, hier von deren Unterseite her. Auch diese Endoprothese 10 zeigt eine erste teilsphärische Vertiefung 12, die als Ersatz für das Acetabulum dient und in einem ersten Abschnitt 13 angeordnet ist. Ebenfalls zu erkennen ist, dass in einem zweiten Abschnitt 14 der Endoprothese 10 eine zweite teilsphärische Vertiefung 15 angeordnet ist. Dieser zweite Abschnitt 14 ist auch bei der gezeigten Endoprothese 10 für eine Anlage an den Beckenknochen des Patienten im Bereich des Os Ilium vorgesehen, die teilsphärische Vertiefung 15 wird dort in eine entsprechend geschaffene Ausnehmung am Os Ilium eingesetzt und mit diesem Beckenabschnitt verbunden.

Auch hier ist um die zweite teilsphärische Vertiefung 15 herum ein über einen weiten Bereich umlaufender, abgeflachter Rand 16 zu erkennen, der sich jedenfalls über den Abschnitt erstreckt, der der ersten teilsphärischen Vertiefung 12 gegenüberliegt. Dieser umlaufende Rand 16 dient, wie auch der umlaufende Rand 6 in dem zuvor gezeigten Beispiel einer glatten Auflage auf dem Beckenknochenmaterial des Os Ilium des Patienten. Auch bei dieser Endoprothese 10 sind von Befestigungsöffnungen 17 durchsetzte Befestigungslaschen 18 angeordnet, und es sind weitere Befestigungsöffnungen 17 in der ersten teilsphärischen Vertiefung 12 zu erkennen. Auch hier dienen die Befestigungslaschen 18 und die Befestigungsöffnungen 17 der Verankerung der Endoprothese 10 am Beckenknochen eines Patienten. Durch Hindurchführen von Knochenschrauben durch die Befestigungsöffnungen 17 und Fixieren derselben im Beckenknochen wird zumindest eine Primärstabilität der Endoprothese 10 erhalten.

Zu erkennen sind auch hier dellenförmige Ausnehmungen 30 auf der konvex gekrümmten Außenseite der zweiten teilsphärischen Vertiefung 15. Derartige dellenförmige Ausnehmungen 30 sind auch auf der in dieser Figur nicht zu erkennenden konkav gekrümmten Innenseite der teilsphärischen Ausnehmung 12 vorgesehen. Schließlich weist auch die Endoprothese 10 einen Durchbruch 19 in der ansonsten einstückig ausgebildeten Fläche und im Bereich zwischen dem ersten Abschnitt 13 und dem zweiten Abschnitt 14 auf.

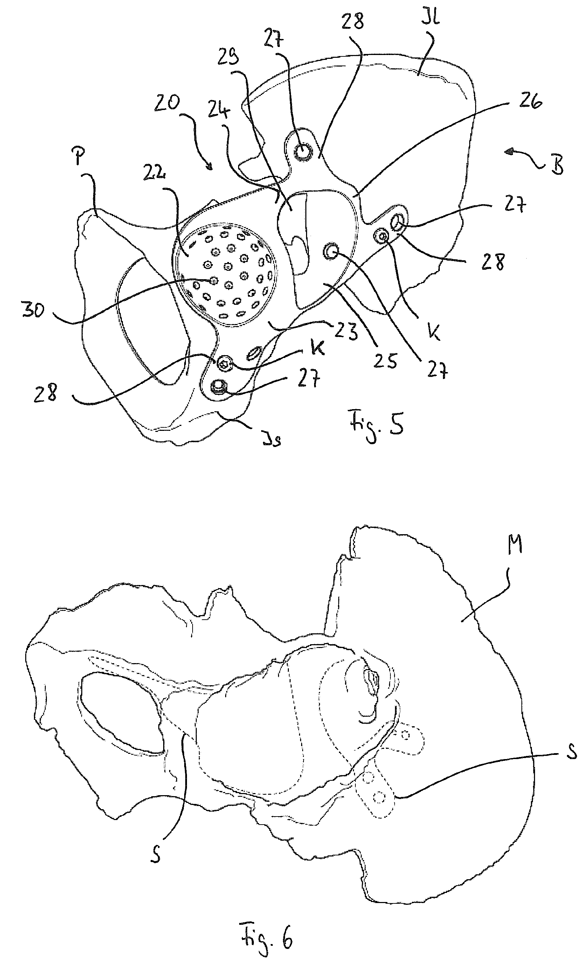

In den Figuren 4 und 5 ist ein drittes Ausführungsbeispiel einer erfindungsgemäßen Endoprothese 20 in einer Implantationsstellung in einem menschlichen Beckenknochen B gezeigt.

Auch die hier in ihrer Implantationsstellung im Beckenknochen B gezeigte Endoprothese 20 hat eine erste teilsphärische Vertiefung 22, die in einem ersten Abschnitt 23 der Endoprothese 20 angeordnet ist. Diese erste teilsphärische Vertiefung 22 bildet in der Endoprothese 20 das Acetabulum des Patienten nach, welches im Verbindungsbereich zwischen Os Ilium I1, Os Pubis P und Os Ischium Is liegt. In einem zweiten Abschnitt 24 der Endoprothese 20, welcher zweiter Abschnitt 24 sich über das Os Ilium I1 erstreckt, ist eine zweite teilsphärische Vertiefung 25 angeordnet. Diese ist in der hier gezeigten Implantationsstellung in einen bei dem Patienten gegebenen Defekt im Os Ilium I1 eingesetzt, wozu im Bereich dieses Defekts in dem Knochen zuvor entsprechend eine Gegenform der teilsphärischen Vertiefung 25 herausgearbeitet wurde. Um die teilsphärische Vertiefung 25 herum, insbesondere auf dem der ersten teilsphärischen Vertiefung 22 gegenüberliegenden Randbereich der zweiten teilsphärischen Vertiefung 25 ist ein glatter und umlaufender Rand 26 gebildet, der auf der Oberfläche des Os Ilium I1 liegt und dort auflastet, auf die Endoprothese 20 wirkende Kräfte dort in den noch gesunden und stabilen Bereich des Os Ilium I1 einleitet.

Mit Befestigungsöffnungen 27 versehene Befestigungslaschen 28 sind auch bei dieser Endoprothese 20 angeordnet. Zu erkennen ist hier, wie die an die zweite teilsphärische Vertiefung 25 angrenzenden Befestigungslaschen 28 über das Os Ilium I1 gelegt sind, wohingegen die an die erste teilsphärische Vertiefung 22 angrenzende Befestigungslasche 28 entlang des Os Ischium Is geführt ist. Zu erkennen sind ferner in Fig. 4 eine Befestigungsöffnung 27 in der ersten teilsphärischen Vertiefung 22 und in Fig. 5 eine Befestigungsöffnung 27 in der zweiten teilsphärischen Vertiefung 25. Weiterhin zu erkennen sind in einzelne der Befestigungsöffnungen 27 repräsentativ eingezeichnete Köpfe von Knochenschrauben K, mit denen die Endoprothese 20 am Beckenknochen B festgelegt ist.

Weiterhin zu sehen ist ein auch bei dieser Endoprothese 20 vorhandener Durchbruch 29 in einem Übergangsbereich zwischen dem ersten Abschnitt 23 und dem zweiten Abschnitt 24.

Wie bei den zuvor gezeigten und beschriebenen Endoprothesen 1 und 10 ist auch hier die konvexe Innenseite der ersten teilsphärischen Vertiefung 22 mit deutlich zu erkennenden dellenartigen Ausnehmungen 30 versehen. Derartige dellenartige Ausnehmungen 30 sind auch auf der hier nicht dargestellten konkaven Außenseite der zweiten teilsphärischen Vertiefung 25 in dem Bereich also, mit dem diese an dem Os Ilium I1 anliegt, vorgesehen.

Wie auch schon die zuvor beschriebenen und gezeigten Endoprothesen 1 und 10 ist auch die Endoprothese 20 einstückig gebildet. Sie besteht in dem gezeigten Ausführungsbeispiel, wie auch in den Fällen der zuvor beschriebenen Ausführungsbeispiele aus einem Metall, insbesondere aus einem Titan oder einer Titanlegierung und ist mit Vorteil hoch poliert.

Die erfindungsgemäße Endoprothese wird typischerweise nicht als fertiges Serienstück hergestellt, sondern individuell für einen Patienten mit weitreichenden Verschleißerscheinungen oder Knochendefekten, z.B. Tumordefekten, im Beckenbereich, welche Defekte sich über das Acetabulum hinaus erstrecken bis hin in das Os Ilium, geplant und gefertigt. Eine Planung erfolgt dabei anhand eines Modells M eines Beckenknochens des Patienten, welches z.B. mit Rapid Prototyping nach Befundaufnahmen mittels bildgebender Verfahren wie z.B. Computertomographie (CT) erstellt worden ist. Im Zuge einer solchen Planung wird ein mit der Planung befasster Medizintechniker zunächst eine geeignete Lage und Orientierung der anzufertigenden Endoprothese auf das Modell M skizzieren, dies z.B. in einer Weise, wie es in der Fig. 6 durch die gestrichelt gezeigten Skizzenlinien S angedeutet ist.

Ausgehend von dem Modell M werden die dann festgelegten Daten und geometrischen Abmessungen für das herzustellende Implantat digitalisiert, und es kann anhand dieser Vorgaben eine passende und individuell abgestimmte Endoprothese gefertigt werden. Diese Fertigung kann beispielsweise in Form eines Gießteils erfolgen, wobei eine individuell für den Patienten bestimmte Gießform hergestellt und darin ein Rohling der Endoprothese gegossen wird. Dieser Rohling wird dann durch weitere Bearbeitung, insbesondere Einbringen der Befestigungsöffnungen der dellenartigen Ausnehmungen sowie eine abschließende Oberflächenbearbeitung, insbesondere eine Hochpolitur, fertig gestellt. Alternativ sind aber auch Vorgehensweisen möglich, bei denen eine Schmiede- oder Gesenkform individuell für den Patienten passend eingerichtet wird und die Endoprothese durch Warm- oder Kaltumformen eines im Wesentlichen ebenen Rohlings in einem Teilfertigprodukt gebildet und entsprechend anschließend fertig bearbeitet wird durch Einbringen der Befestigungsbohrungen, Ausbilden der dellenartigen Ausnehmungen sowie eine abschließende Oberflächenbearbeitung.

### Bezugszeichenliste

- 1: Endoprothese
- 2: teilsphärische Vertiefung
- 3: Abschnitt
- 4: Abschnitt
- 5: teilsphärische Vertiefung
- 6: Rand
- 7: Befestigungsöffnung
- 8: Befestigungslasche
- 9: Durchbruch
- 10: Endoprothese
- 12: teilsphärische Vertiefung
- 13: Abschnitt
- 14: Abschnitt
- 15: teilsphärische Vertiefung
- 16: Rand
- 17: Befestigungsöffnung
- 18: Befestigungslasche
- 19: Durchbruch
- 20: Endoprothese
- 22: teilsphärische Vertiefung
- 23: Abschnitt
- 24: Abschnitt
- 25: teilsphärische Vertiefung
- 26: Rand
- 27: Befestigungsöffnung
- 28: Befestigungslasche
- 29: Durchbruch
- 30: dellenartige Ausnehmung

- B: Beckenknochen
- Il: Os Ilium
- Is: Os Ischium
- K: Knochenschraube
- M: Modell
- P: Os Pubis
- S: Skizzenlinie

## Patentansprüche

1. Endoprothese für den Teilersatz des menschlichen Beckenknochens (B) im Bereich des Acetabulum sowie des Os Ilium (Il) mit einem ersten Abschnitt (3; 13; 23), der eine erste teilsphärische Vertiefung (2; 12; 22) aufweist, die als Ersatz für das Acetabulum dient, sowie mit einem zweiten Abschnitt (4; 14; 24) für die Anlagen am Os Ilium (Il), **dadurch gekennzeichnet, dass** sich der zweite Abschnitt (4; 14; 24) ausgehend von dem ersten Abschnitt (3; 13; 23) über den Rand der ersten teilsphärischen Vertiefung (2; 12; 22) abgeflacht weiter erstreckt und einstückig mit dem ersten Abschnitt (3; 13; 23) verbunden ist und dass in dem zweiten Abschnitt (4; 14; 24) eine zweite teilsphärische Vertiefung (5; 15; 25) in gleicher Richtung eingebracht ist wie die erste teilsphärische Vertiefung (2; 12; 22).

2. Endoprothese nach Anspruch 1, **gekennzeichnet durch** einen zumindest auf der der ersten teilsphärischen Vertiefung (2; 12; 22) gegenüberliegenden Seite der zweiten teilsphärischen Vertiefung (5; 15; 25) an dieser angeformten und im Winkel abstehenden verbreiterten und abgeflachten Rand (6; 16; 26).

3. Endoprothese nach einem der vorhergehenden Ansprüche, **dadurch gekennzeichnet, dass** in dem ersten (3; 13; 23) und/oder in dem zweiten (4; 14; 24) Abschnitt einstückig angeformte, sich nach außen erstreckende Laschen (8; 18; 28) vorgesehen sind mit jeweils wenigstens einer Durchlassöffnung (7; 17; 27) zum Durchführen von Befestigungsmitteln (K).

4. Endoprothese nach einem der vorhergehenden Ansprüche, **dadurch gekennzeichnet, dass** in der ersten teilsphärischen Vertiefung (2; 12; 22) wenigstens eine Durchlassöffnung (7; 17; 27) zum Durchführen eines Befestigungsmittels (K) angeordnet ist.

5. Endoprothese nach einem der vorhergehenden Ansprüche, **gekennzeichnet durch** einen in einem Übergangsbereich zwischen dem ersten (3; 13; 23) und dem zweiten (4; 14; 24) Abschnitt und zwischen der ersten (2; 12; 22) und der zweiten (5; 15; 25) teilsphärischen Vertiefung angeordneten, ansonsten vom Material der einstückig gebildeten Endoprothese (1; 10; 20) umgebenen Durchbruch (9; 19; 29).

6. Endoprothese nach einem der vorhergehenden Ansprüche, **dadurch gekennzeichnet, dass** im Bereich der ersten teilsphärischen Vertiefung (2; 12; 22) auf der konkav gekrümmten Seite des Materials dellenartige Ausnehmungen (30) angeordnet sind.

7. Endoprothese nach einem der vorhergehenden Ansprüche, **dadurch gekennzeichnet, dass** im Bereich der zweiten teilsphärischen Vertiefung (5; 15; 25) auf der konvex gekrümmten Seite des Materials dellenartige Ausnehmungen angeordnet sind.

8. Endoprothese nach einem der vorhergehenden Ansprüche, **dadurch gekennzeichnet, dass** in dem Bereich, in dem sich an die erste teilsphärische Ausnehmung (2; 12; 22) an deren Rand der zweite Abschnitt (4; 14; 24) anschließt, eine das kraniale Pfannendach nachbildende Struktur gegeben ist.

9. Endoprothese nach einem der vorhergehenden Ansprüche, **dadurch gekennzeichnet, dass** sie aus einem, vorzugsweise hoch polierten, Metall, insbesondere Titan oder einer Titanlegierung, besteht.

10. Endoprothese nach einem der vorhergehenden Ansprüche, **dadurch gekennzeichnet, dass** sie durch Gießen und weiteres Bearbeiten des Gussteils hergestellt ist.

11. Endoprothese nach einem der Ansprüche 1 bis 9, **dadurch gekennzeichnet, dass** sie durch Kalt- oder Warmumformen aus einem im Wesentlichen flachen Ausgangswerkstück und weiteres Bearbeiten des so entstandenen Formteils hergestellt ist.

## Claims

1. Endoprosthesis for the partial replacement of the human pelvic bone (B) in the area of the acetabulum and of the ilium (Il), with a first portion (3; 13; 23), which has a first partially spherical recess (2; 12; 22) serving as a replacement for the acetabulum, and with a second portion (4; 14; 24) for bearing on the ilium (Il), **characterized in that** the second portion (4; 14; 24), starting from the first portion (3; 13; 23), extends flattened over the edge of the first partially spherical recess (2; 12; 22) and is integrally connected to the first portion (3; 13; 23), and **in that** a second partially spherical recess (5; 15; 25) is introduced in the second portion (4; 14; 24), in the same direction as the first partially spherical recess (2; 12; 22).

2. Endoprosthesis according to Claim 1, **characterized by** a widened and flattened edge (6; 16; 26) integrally formed at least on the side of the second partially spherical recess (5; 15; 25) opposite the first partially spherical recess (2; 12; 22) and protruding at an angle.

3. Endoprosthesis according to either of the preceding claims, **characterized in that** outwardly extending tabs (8; 18; 28) integrally formed in the first portion (3; 13; 23) and/or in the second portion (4; 14; 24) are provided, in each case with at least one through-opening (7; 17; 27) for the passage of securing means (K).

4. Endoprosthesis according to one of the preceding claims, **characterized in that** at least one through-opening (7; 17; 27) for the passage of a securing means (K) is arranged in the first partially spherical recess (2; 12; 22).

5. Endoprosthesis according to one of the preceding claims, **characterized by** an aperture (9; 19; 29) arranged in a transition area between the first portion (3; 13; 23) and the second portion (4; 14; 24) and between the first partially spherical recess (2; 12; 22) and the second partially spherical recess (5; 15; 25) and otherwise surrounded by the material of the integrally formed endoprosthesis (1; 10; 20).

6. Endoprosthesis according to one of the preceding claims, **characterized in that** dimple-like indentations (30) are arranged in the area of the first partially spherical recess (2; 12; 22), on the concave side of the material.

7. Endoprosthesis according to one of the preceding claims, **characterized in that** that dimple-like indentations (30) are arranged in the area of the second partially spherical recess (5; 15; 25), on the convex side of the material.

8. Endoprosthesis according to one of the preceding claims, **characterized in that** a structure simulating the cranial roof of the acetabulum is provided in the area in which the second portion (4; 14; 24) adjoins the edge of the first partially spherical recess (2; 12; 22).

9. Endoprosthesis according to one of the preceding claims, **characterized in that** it is made of a preferably highly polished metal, in particular titanium or a titanium alloy.

10. Endoprosthesis according to one of the preceding claims, **characterized in that** it is produced by casting and further processing of the cast part.

11. Endoprosthesis according to one of Claims 1 to 9, **characterized in that** it is produced by cold or hot working from a substantially flat starting workpiece and by further processing of the resulting shaped part.

## Revendications

1. Endoprothèse pour le remplacement partiel de l'os du bassin (B) humain au niveau de l'acétabulum ainsi que de l'ilium (Il), présentant une première section (3 ; 13 ; 23), qui présente un premier creux (2 ; 12 ; 22) partiellement sphérique qui sert de substitut pour l'acétabulum, ainsi qu'une deuxième section (4 ; 14 ; 24) pour le contact avec l'ilium (Il), **caractérisée en ce que** la deuxième section (4 ; 14 ; 24) s'étend de manière aplatie, partant de la première section (3 ; 13 ; 23), au-delà du bord du premier creux (2 ; 12 ; 22) partiellement sphérique et est assemblée d'une seule pièce avec la première section (3 ; 13 ; 23) et **en ce qu'**un deuxième creux (5 ; 15 ; 25) partiellement sphérique est réalisé dans la deuxième section (4 ; 14 ; 24) dans le même sens que le premier creux (2 ; 12 ; 22) partiellement sphérique.

2. Endoprothèse selon la revendication 1, **caractérisée par** un bord (6 ; 16 ; 26) élargi et aplati formé au moins sur le côté du deuxième creux (5 ; 15 ; 25) partiellement sphérique opposé au premier creux (2 ; 12 ; 22) partiellement sphérique et formant un angle par rapport audit deuxième creux.

3. Endoprothèse selon l'une quelconque des revendications précédentes, **caractérisée en ce que** la première section (3 ; 13 ; 23) et/ou la deuxième section (4 ; 14 ; 24) présente des pattes (8 ; 18 ; 28) formées d'une seule pièce, s'étendant vers l'extérieur présentant à chaque fois au moins une ouverture de passage (7 ; 17 ; 27) pour le passage de moyens de fixation (K).

4. Endoprothèse selon l'une quelconque des revendications précédentes, **caractérisée en ce qu'**au moins une ouverture de passage (7 ; 17 ; 27) pour le passage d'un moyen de fixation (K) est disposée dans le premier creux (2 ; 12 ; 22) partiellement sphérique.

5. Endoprothèse selon l'une quelconque des revendications précédentes, **caractérisé par** un percement (9 ; 19 ; 29) disposé dans une zone de transition entre la première section (3 ; 13 ; 23) et la deuxième section (4 ; 14 ; 24) et entre le premier creux (2 ; 12 ; 22) partiellement sphérique et le deuxième creux (5 ; 15 ; 25) partiellement sphérique, entouré pour le reste par du matériau de l'endoprothèse (1 ; 10 ; 20) formée d'une seule pièce.

6. Endoprothèse selon l'une quelconque des revendications précédentes, **caractérisée en ce que** des évidements de type cuvette sont disposés au niveau du premier creux (2 ; 12 ; 22) partiellement sphérique sur le côté incurvé de manière concave du matériau.

7. Endoprothèse selon l'une quelconque des revendications précédentes, **caractérisée en ce que** des évidements de type cuvette sont disposés au niveau du deuxième creux (5 ; 15 ; 25) partiellement sphérique sur le côté incurvé de manière convexe du matériau.

8. Endoprothèse selon l'une quelconque des revendications précédentes, **caractérisée en ce qu'**une structure imitant une cupule acétabulaire crâniale existe dans la zone dans laquelle la deuxième section (4 ; 14 ; 24) se raccorde au bord du premier évidement (2 ; 12 ; 22) partiellement sphérique.

9. Endoprothèse selon l'une quelconque des revendications précédentes, **caractérisée en ce qu'**elle est formée par un métal, de préférence hautement poli, en particulier le titane ou un alliage de titane.

10. Endoprothèse selon l'une quelconque des revendications précédentes, **caractérisée en ce qu'**elle est fabriquée par coulée et par usinage consécutif de la pièce coulée.

11. Endoprothèse selon l'une quelconque des revendications 1 à 9, **caractérisée en ce qu'**elle est fabriquée par formage à froid ou à chaud à partir d'une pièce de départ essentiellement plate et par usinage consécutif de la pièce façonnée ainsi formée.
